# NEUE EUROPÄISCHE PATENTSCHRIFT

(11) **EP 1 034 777 B2**
(45) Veröffentlichungstag und Bekanntmachung des Hinweises auf die Entscheidung über den Einspruch: **14.08.2013**
(45) Hinweis auf die Patenterteilung: 19.11.2003
(21) Anmeldenummer: 00102013.0
(22) Anmeldetag: 02.02.2000
(51) Int. Cl.: A61K 8/22

(54) **Lipophiles Mittel zum Entfärben oder Blondieren von Haaren**
Lipophilic hair decolorating or bleaching composition
Composition lipophilique décolorante pour cheveux

(30) Priorität: 05.03.1999 DE 19909661
(43) Veröffentlichungstag der Anmeldung: 13.09.2000
(73) Patentinhaber: Wella GmbH, 65825 Schwalbach am Taunus (DE)
(72) Erfinder: Lauscher, Dirk, Dr., 64342 Seeheim-Jugenheim (DE); Deutz, Herbert, Dr., 64673 Zwingenberg (DE); Hess, Gabriele, 64390 Erzhausen (DE); Kreher, Helga, 64839 Münster (DE); Braun, Petra, 64839 Münster (DE); Schmidt, Jörg, 63263 Neu-Isenburg (DE)
(74) Vertreter: Boubel, Thomas

(56) Entgegenhaltungen:
- EP-A- 0 560 088
- EP-A1- 0 778 020
- EP-A1- 1 036 558
- EP-A2- 0 882 444
- WO-A-94/16672
- DE-A- 3 814 356
- DE-A- 4 026 235
- DE-A- 19 545 853
- DE-C- 19 723 538
- GB-A- 466 172
- US-A- 4 170 637
- US-A- 5 698 186
- ZIOLKOWSKY, BERND: "Kosmetische Gele: Aufbau und neuere Entwicklungen" SÖFW-JOURNAL, Bd. 120, Nr. 5, Mai 1994 (1994-05), Seiten 280-284, XP000914570

## Beschreibung

Gegenstand der vorliegenden Erfindung ist ein aus zwei Komponenten erhältliches Mittel zum Entfärben oder Blondieren (Bleichen) von Haaren, insbesondere menschlichen Haaren.

Zum Entfärben oder Blondieren von Haaren werden üblicherweise oxidierende Zubereitungen verwendet, welche durch Auflösen eines sogenannten Blondierpulvers (Pulvergemisch aus Alkalisalzen und anorganischen Persalzen, wie zum Beispiel Natrium- oder Ammoniumpersulfat) in einer wäßrigen Wasserstoffperoxidlösung erhalten werden.

Die Verwendung derartiger Blondierpulver, welche notwendigerweise aus mehreren Bestandteilen zusammengesetzt sind, bringt jedoch eine Vielzahl von Nachteilen mit sich. So kommt es durch die Verwendung von Rohstoffen mit unterschiedlicher Dichte häufig während des Transportes oder der Lagerung zur Trennung der unterschiedlichen Pulverbestandteile, wobei sich die schweren Pulverbestandteile im unteren Teil und die leichteren Pulverbestandteile im oberen Teil des Behältnisses ansammeln. Diese Entmischung hat zur Folge, daß die gleiche Pulvermenge je nach ihrem Entnahmeort eine unterschiedliche chemische Zusammensetzung und somit auch eine unterschiedliche Blondierwirkung aufweisen kann. Um dieser Entmischung entgegenzuwirken, ist es erforderlich, vor jeder Pulverentnahme das Pulver gründlich zu schütteln, was der Verwender in der Regel jedoch nicht tut. Eine Entmischung kann auch durch den Einsatz von Pulver-gemischen mit sehr kleiner Korngröße verhindert werden. Dies hat jedoch den Nachteil, daß solche Pulvergemische - insbesondere beim Öffnen der Behältnisse, bei der Entnahme des Pulvers oder beim Vermischen mit der Wasserstoffperoxidlösung - zu einer starken Staubentwicklung neigen, was zu einer Reizung der Atmungsorgane führen kann. Weiterhin besitzen derartige Pulvergemische aufgrund der geringen Korngröße eine große Oberfläche, wodurch eine Aufnahme von Feuchigkeit beim Öffnen und Schließen des Behältnisses und damit eine Verringerung der Blondierwirkung infolge der Desaktivierung des Sauerstoffträgers begünstigt wird.

Die Zubereitung der gebrauchsfertigen Mischung erfolgt zum einen durch Verrühren der Bestandteile in einer Schale, zum anderen durch Vermischen in einer Anschüttelflasche, wobei insbesondere das Anschütteln oftmals mit einer lästigen Staubentwicklung beim Einfüllen der Komponenten in die Schüttelflasche verbunden ist.

Es wurden bereits zahlreiche Versuche unternommen, dieses Problem zu lösen.
So wird in der DE-OS 40 26 235 vorgeschlagen, anstelle eines Blondierpulvers eine Mischung, bestehend aus einem Granulat der anorganischen Persulfate und einem Granulat der übrigen Bestandteile des Blondiermittels, zu verwenden. Hierdurch kann zwar das Problem der Staubentwicklung behoben werden, das Problem der Entmischung kann auf diesem Wege jedoch nicht gelöst werden, da es technisch äußerst schwierig ist, Einzelgranulate mit identischer und konstanter Komgröße oder Schüttgewicht herzustellen. Weiterhin kann es aufgrund der unterschiedlichen Löslichkeit der einzelnen Granulate zu einer Beeinträchtigung der Blondierwirkung kommen. Es erscheint zudem auch aus ökonomischen Gesichtspunkten wenig sinnvoll, anstelle eines einzelnen Granulates eine Mischung aus mehreren Granulaten herzustellen.In der EP-PS 0 560 088 wird ein pulverförmiges Mittel zum Blondieren von Haaren beschrieben, bei dem zur Verhinderung der Staubbildung ein Öl oder flüssiges Wachs zugesetzt wird. Aber auch hierdurch kann eine völlige Staubfreiheit nicht erreicht werden. Zudem ergibt sich durch den Wassergehalt der eingesetzten pulverförmigen Rohstoffe und die kompakte Pulverform eine Desaktivierung der Sauerstoffträger, wodurch das Produkt instabil wird und seine Bleichwirkung verliert. Weiterhin sind derartige Blondiermittel aufgrund ihres spezifischen Gewichtes und ihres hydrophoben Charakters für die Verwendung in der Auftrageflasche ungeeignet, da das Pulver in der Wasserstoffperoxidlösung nach unten sinkt und nicht ausreichend benetzt wird, wodurch eine inhomogene Mischung mit einem hohen Anteil an ungelöstem Pulver erhalten wird, durch die die Austrittsdüse der Auftrageflasche verstopft wird. Der Zusatz von Tensiden zur Verbesserung der Löslichkeit des Pulvers ist ebenfalls problematisch, da hierdurch die Lagerfähigkeit des Pulvers beeinträchtigt wird.
In der DE-OS 38 14 356 sowie der US-PS 4 170 637 werden breiartige Zweikomponenten-Zubereitungen zur Anfertigung einer auftragefähigen, breiartigen Zubereitung zum Bleichen von Humanhaaren beschrieben, bei denen die pulverförmigen Bestandteile in eine hydrophobe Basis, bestehend aus Ölen und Wachsen, eingearbeitet sind, so daß eine Paste resultiert. Diese Suspensionen haben den Nachteil, daß sie leicht zu Phasentrennungen neigen, die sich als Ölabscheidung bemerkbar machen. Um dieses zu verhindern, wird ein absorbierendes Mittel wie zum Beispiel disperse Kieselsäure zugesetzt, wodurch wiederum die Paste sehr fest wird. In der DE 197 23 538 C werden breiartige Zweikomponenten-Zubereitungen zur Anfertigung einer auftragefähigen, breiartigen Zubereitung zum Bleichen von Humanhaaren beschrieben, welche neben den üblichen Bestandteilen von Blondierungen eine bestimmte Verdickerkombination, bestehend aus einem Acrylsäurepolymer und mindestens einem Polymer aus der Gruppe der Cellulosen, Alginate und Polysaccharide, mindestens einem Mineralöl, mindestens einem flüssigen, langkettigen, hydrophoben Fettsäureester und mindestens einem wachsförmigen, langkettigen, hydrophoben Fettsäureester und/oder synthetischen Bienenwachsersatzstoff enthalten. Diese Mittel sind jedoch in Bezug auf ihr Verhalten bei hohen Temperaturen nicht in jeder Hinsicht befriedigend.
Aus der DE 195 45 853 A sind ebenfalls Blondiermittelsuspensionen bekannt, welche neben den üblichen Bestandteilen von Blondierungen eine bestimmte Verdickerkombination, bestehend aus einem Acrylsäurepolymer und mindestens einem Polymer aus der Gruppe der Cellulosen, Alginate und Polysaccharide und mindestens ein Öl oder Wachs enthalten. In der WO 94/16672 A werden pulverförmige Blondiermittel beschrieben, welche neben den üblichen Bestandteilen von Blondierungen einen Verdicker und eine Siliciumverbindung enthalten.
In der US 4 170 637 A werden unter anderem Bleichmittel beschrieben, welche neben den üblichen Bestandteilen von Blondierungen Öle/Wachse, Xanthan Gum und Siliciumdioxid enthalten. Weiterhin ist aus der DE 38 14 356 A ein pastenförmiges Bleichmittel bekannt, das neben den üblichen Bestandteilen von Blondierungen Öle/Wachse, disperse Kieselsäure und ein spezielles Smektit-Derivat enthält.

Es bestand daher die Aufgabe, ein lagerstabiles, nicht-staubendes Mittel zum Entfärben oder Blondieren von Haaren zur Verfügung zu stellen, welches vor Gebrauch durch einfaches Schütteln oder Anrühren mit einer Oxidationsmittelzubereitung vermischt wird und neben seiner absolut staubfreien Darreichungs- und Anwendungsform eine sehr gute Blondierwirkung bei gleichzeitiger guter Lagerstabilität zwischen 5 und 45°C gewährleistet, ohne die hervorragende, cremeartige Konsistenz und damit die hervorragenden Gebrauchseigenschaften unter Kälteeinfluß bzw. Wärmeeinfluß zu verlieren .

Überraschend wurde nunmehr gefunden, daß die vorstehend beschriebene Aufgabe durch Verwendung eines pastenförmigen Blondiermittels basierend auf einer speziellen, neuartigen Kombination von Verdickungsmitteln gelöst werden kann, wobei der Zusatz von Emulgatoren nicht erforderlich ist.

Gegenstand der vorliegenden Erfindung ist daher ein Mittel zum Entfärben oder Blondieren von Haaren, insbesondere menschlichen Haaren, welches unmittelbar vor der Anwendung mit einer wäßrigen Oxidationsmittelzubereitung vermischt wird und dadurch gekennzeichnet ist, daß es in Form einer Blondiermittelsuspension vorliegt und eine Kombination aus
(a) 20 bis 50 Gewichtsprozent mindestens einer organisch lipophilen Verbindung aus der Gruppe der pflanzlichen und tierischen Fette, Öle und Wachse, der Paraffinkohlenwasserstoffe, der höheren Alkohole und Ether, der aliphatischen und aromatischen Ester sowie der Silikonöle;
(b) 0,1 bis 40 Gewichtsprozent mindestens eines anorganischen oder organischen Verdickers mit lipophilem Charakter, der mit der lipophilen Verbindung ein Oleogel bzw. Lipogel ausbildet, welcher ausgewählt ist aus, Erdalkalicarboxylaten, Aluminiumcarboxylaten, Copolymerisaten von Alkenen, vemetzten organischen Polymeren und lipophilisierten Schichtsilikaten, oder Mischungen dieser Verdicker;
(c) 0,1 bis 40 Gewichtsprozent mindestens eines anorganischen oder organischen Verdickers mit hydrophilem Charakter, welcher ausgewählt ist aus Polymeren aus der Gruppe der Cellulosen, Alginate, Polysaccharide und Acrylsäuren;
(d) 10 bis 55 Gewichtsprozent mindestens eines anorganischen Persalzes;
(e) 15 bis 35 Gewichtsprozent mindestens eines alkalisch reagierenden Salzes;
sowie gegebenenfalls Hilfsstoffen und Zusatzstoffen enthält und einen Wassergehalt von maximal 3% aufweist.

Als organisch lipophile Verbindungen eignen sich insbesondere Pflanzenöle wie zum Beispiel Jojobaöl; Vaseline; flüssige Paraffine, beispielsweise Paraffinum Perliquidum und Paraffinum Subliquidum; Siliconöle; flüssige, langkettige, hydrophobe Fettsäureester, beispielsweise Octylpalmitat, Isocetylpalmitat, Isopropylpalmitat und Octylstearat; wachsförmige, langkettige, hydrophobe Fettsäureester und/oder synthetische Wachsersatzstoffe, beispielsweise natürliches oder synthetisches Bienenwachs (zum Beispiel Lipowachs 6138G® der Firma Lipo Chemicals), C18- bis C36-Fettsäuren (zum Beispiel Synchrowachs AW1C® der Firma Croda Chemicals Ltd.), C-10 bis C36-Fettsäuretriglyceride, wie zum Beispiel Octansäure/Dodecansäure-Triglycerid, hydrierte Kokosfettsäureglyceride (zum Beispiel Softisan 100® der Firma Hüls AG), Glyceryltribehenat (beispielsweise Synchrowachs HRC® der Firma Croda Chemicals Ltd.), Fettsäureestergemische (beispielsweise Cutina BW® der Firma Henkel KGaA), sowie deren Mischungen. Besonders bevorzugt ist hierbei die Verwendung von Jojobaöl, Fettsäureestern, Paraffinöl, Kombinationen aus Fettsäureestem und Paraffinöl sowie Kombinationen aus Fettsäureestern und/oder Paraffinöl mit Vaseline.

Die lipophilen Verbindungen werden, bezogen auf die Gesamtmenge der Blondiermittelsuspension, in einer Gesamtmenge von 20 bis 50 Gewichtsprozent, eingesetzt.

Als lipophile anorganische oder organische Verdicker sind insbesondere, Aluminium/Magnesiumhydroxystearat oder Magnesiumstearat, Aluminiummonostearat, Aluminiummonodistearat und/oder Aluminiumtristearat, Ethylen/Propylen-Copolymere, Benzyldimethyl-stearylammonium-Hectorit (beispielsweise Bentone 28® der Fa. Rheox), sowie Gemische dieser lipophilen Verdicker zu nennen, wobei, Erdalkalistearate, Aluminiumstearate und Aluminium/Magnesiumhydroxystearat, und insbesondere Magnesiumstearate und Aluminiumstearate, bevorzugt sind.

Die lipophilen Verdicker oder deren Gemische bilden bei Auflösung in den beschriebenen lipophilen Verbindungen ein Oleogel bzw. Lipogel aus. Das Auflösen des lipophilen Verdickers in der lipophilen Komponente kann hierbei durch Erhitzen oder durch die Verwendung von Lösungsvermittlern, wie zum Beispiel Propylencarbonat, unterstützt werden.
Ebenfalls ist es möglich, fertige Gemische von lipophilen Verdickem und lipophilen Verbindungen, beispielsweise Brooks Gel® der Fa. Brooks Industries, die Bentone Gel®-Typen der Fa. Rheox, die Myglyol Gel®- und Softisan Gel®-Typen der Fa. Hüls AG sowie die Gilugel®-Typen der Fa. BK Giulini Chemie, zu verwenden, wobei die gewünschte Konsistenz durch Zugabe weiterer Öle individuell eingestellt werden kann.

Bezogen auf die Gesamtmenge der Blondiermittelsuspension werden die oleo- bzw. lipogelbildenden Verdicker in einer Gesamtmenge von etwa 0,1 bis 40 Gewichtsprozent, vorzugsweise von 0,2 bis 20 Gewichtsprozent eingesetzt, wobei eine Einsatzmenge von 0,5 bis 15 Gewichtsprozent besonders bevorzugt ist.

Als hydrophile anorganische oder organische Verdicker können vorzugsweise Methylcellulosen, Ethylcellulosen, Hydroxyethylcellulosen, Methylhydroxyethylcellulosen, Methylhydroxypropylcellulosen, Carboxymethylcellulosen, Alginsäure, Natriumalginat, Ammoniumalginat, Calciumalginat, Gummi Arabicum, Guar Gum oder Xanthan Gum, oder Acrylsäurepolymere mit einem Molekulargewicht von etwa 1.250.000 bis 4.000.000, alleine oder in Kombination miteinander, verwendet werden, wobei die Verwendung von anquellverzögernden Methylhydroxyethylcellulosen, einer Kombination von Alginaten mit Polysacchariden oder/und Cellulosen oder einer Kombination von Alginaten und/oder Cellulosen mit Acrylsäurepolymeren besonders bevorzugt ist.

Bezogen auf die Gesamtmenge der Blondiermittelsuspension werden die hydrophilen Verdicker in einer Gesamtmenge von etwa 0,1 bis 40 Gewichtsprozent, vorzugsweise von 0,2 bis 20 Gewichtsprozent, und insbesondere von 0,5 bis 15 Gewichtsprozent eingesetzt.

Als anorganische Persalze werden vorzugsweise Erdalkaliperoxide und anorganische Persulfate, beispielsweise Ammoniumpersulfat und Alkalipersulfate wie Natriumpersulfat oder Kaliumpersulfat, oder Mischungen dieser anorganischen Persalze eingesetzt, wobei die Persalze bezogen auf die Gesamtmenge der Blondiermittelsuspension in einer Gesamtmenge von vorzugsweise 10 bis 55 Gewichtsprozent, insbesondere von 20 bis 55 Gewichtsprozent, eingesetzt werden.

Als alkalisch reagierende Salze werden in wäßriger Lösung alkalisch reagierende Alkalimetallsalze oder Erdalkalimetallsalze, beispielsweise Natriumcarbonat, Natriumhydrogencarbonat, Magnesiumcarbonat, Ammoniumcarbonat, Ammoniumhydrogencarbonat, Natriumsilikate oder Mischungen dieser Salze, verwendet, wobei diese Salze bezogen auf die Gesamtmenge der Blondiermittelsuspension in einer Gesamtmenge von 15 bis 35 Gewichtsprozent, eingesetzt werden.

Zusätzlich kann die cremeförmige Blondiermittelsuspension weitere für derartige Zubereitungen übliche Zusatzstoffe, wie zum Beispiel Pflegestoffe, Siliciumdioxid, Titandioxid, Chelatisierungsmittel für Schwermetallionen, beispielsweise Ethylendiamintetraessigsäure, Anfärbestoffe, beispielsweise Ultramarinfarbstoffe, oder Parfüme, enthalten, wobei diese Zusatzstoffe in den für solche Mittel üblichen Einsatzmengen eingesetzt werden; beispielsweise die Pflegestoffe, das Siliciumdioxid und das Chelatisierungsmittel jeweils in einer Menge von 0,01 bis 3 Gewichtsprozent, und die Anfärbestoffe und Parfüme jeweils in einer Menge von 0,01 bis 2 Gewichtsprozent.

Vorzugsweise enthält die erfindungsgemäße Blondiermittelsuspension keine Tenside oder Emulgatoren und ist wasserfrei, wobei ein Wassergehalt von maximal bis zu 3 Gewichtsprozent jedoch zulässig ist.

Vor der Anwendung wird die erfindungsgemäße Blondiermittelsuspension mit der Oxidationsmittelzubereitung zu einem auftragefähigen Blondierbrei vermischt, wobei dieses Vermischen in einer Schale oder durch Anschütteln in einer Auftrageflasche erfolgen kann. Als Oxidationsmittelzubereitung kann insbesondere eine Wasserstoffperoxid enthaltende wäßrige Lösung oder Öl-in-Wasser-Emulsion (insbesondere eine 6- bis 12-prozentige Wasserstoffperoxid-lösung oder Wasserstoffperoxid-emulsion) verwendet werden. Es ist jedoch auch möglich, anstelle von Wasserstoffperoxid Wasserstoffperoxid-abspaltende Addukte, beispielsweise Harnstoffperoxid oder Melaminperhydrat, zu verwenden.

Das Mischungsverhältnis von Blondiermittelsuspension zu Oxidationsmittelzubereitung beträgt vorzugsweise 2:1 bis 1:8, insbesondere 1:1 bis 1:5.

Das nach dem Vermischen mit der Oxidationsmittelzubereitung erhaltene gebrauchsfertige Mittel zum Entfärben oder Blondieren von Haaren weist einen pH-Wert von etwa 7,5 bis 11, insbesondere von 8 bis 9,5, auf.

Das gebrauchsfertige Mittel wird auf das Haar gleichmäßig aufgetragen und nach einer Einwirkungszeit von 15 bis 60 Minuten bei Raumtemperatur (20-25°C) beziehungsweise von 10 bis 50 Minuten bei Wäremeinwirkung (30-50°C) mit Wasser ausgespült.

Die cremeförmige Blondiermittelsuspension kann je nach ihrer Viskosität in Tuben, Sachets oder Tiegel abgefüllt werden. Neben der anwendungsfreundlichen Produktviskosität über einen großen Temperaturbereich und der leichten Anmischbarkeit mit dem Oxidationsmittel zeichnet sich das erfindungsgemäße Mittel durch eine hervorragende Lagerstabilität, Auftragefähigkeit, Verteilbarkeit und Haftung am Haar sowie ein breites Anwendungsspektrum aus. Im Vergleich zu herkömmlichen Blondiermitteln erreicht das erfindungsgemäße Mittel eine bessere Aufhellungen bei geringerem Gehalt an aktiven Bleichwirkstoffen. Durch die emulgierenden Eigenschaften der gelbildenden Komponenten läßt sich das erfindungsgemäße Blondiermittel sehr leicht mit Wasser wieder rückstandsfrei aus dem Haar ausspülen.

Die nachfolgenden Beispiele sollen den Gegenstand näher erläutern.

### Beispiele

### Beispiel 1: Blondiermittel

| Cremeförmige Blondiermittelsuspension | |
|---|---|
| 8,0 g | Isododecan/Ethylen-Mixed-Copolymer (Brooks Gel® der Brooks Industries Inc.) |
| 8,0 g | Isopropylpalmitat |
| 16,0 g | Jojobaöl |
| 4,0 g | C10-C18 Fettsäure-Triglycerid (Nesatol® der Fa. Vevy) |
| 24,2 g | Natriummetasilikat |
| 4,0 g | Natriumalginat |
| 11,3 g | Diammoniumpersulfat |
| 22,5 g | Dikaliumpersulfat |
| 1,0 g | Zinkstearat |
| 1,0 g | Ethylendiamintetraessigsäure-Dinatriumsalz |
| 100,0 g | |

Zur Herstellung der cremeförmigen Blondiermittelsuspension werden zunächst die flüssigen Komponenten mit dem lipophilen Brooks Gel® (Oleogel) bei Raumtemperatur homogen vermischt und anschließend die vorgemischten festen Rohstoffe eingetragen. Es wird darauf geachtet, daß eine homogene Verteilung der Feststoffe in der Lipogelmatrix erfolgt.

### Anwendung

25 g der vorstehend beschriebenen Blondiermittelsuspension 1 werden mit 75 g einer 6prozentigen wäßrigen Wasserstoffperoxidlösung der folgenden Zusammensetzung

| | |
|---|---|
| 6,0 g | Wasserstoffperoxid |
| 2,0 g | Cetylstearylalkohol |
| 0,2 g | Lanolinalkohol |
| 0,1 g | Phosphorsäure (85-prozentig) |
| 91,7 g | Wasser |
| 100,0 g | |

in einer Auftrageflasche 10 bis 15 Sekunden geschüttelt. Anschließend wird das Blondiermittel mittels einer Auftrageflasche gleichmäßig auf das aufzuhellende Haar aufgetragen. Nach einer Einwirkungszeit von 30 Minuten bei Raumtemperatur (20 bis 30° C) wird das Haar mit warmem Wasser gründlich ausgespült und getrocknet.
Der Aufhellungsgrad beträgt 3 Tonstufen und kann durch Verlängerung der Einwirkungszeit um 20 Minuten um 1 bis 2 Tonstufen erhöht werden.

### Beispiel 2: Blondiermittel

| Cremeförmige Blondiermittelsuspension | |
|---|---|
| 1,9 g | Dimethyldistearyl-ammonium-hectorit (Quaternium-18 Hectorite; Bentone® 38CE der Rheox Inc.) |
| 0,5 g | Propylencarbonat |
| 34,1 g | Octylstearat |
| 23,0 g | Dinatriumpersulfat |
| 17,0 g | Dikaliumpersulfat |
| 20,0 g | Natriummetasilikat |
| 1,5 g | Xanthan Gum |
| 1,5 g | Acrylsäurepolymer (Synthalen® K der Fa. 3V-Sigma) |
| 0,5 g | Ethylendiamintetraessigsäure-Dinatriumsalz |
| 100,0 g | |

Zur Herstellung dieser cremeförmigen Blondiermittelsuspension wird das organisch-modifizierte lipophile Schichtsilikat (Bentone® 38CE) mit Propylencarbonat benetzt, das Octylstearat zugegeben und das Gemisch anschließend 3 Minuten lang mit einem Rotor-Stator-System bei 20.000 U/min bei Raumtemperatur homogenisiert. Die vorgemischten festen Rohstoffe werden anschließend in das auf diese Weise hergestellte Lipogel eingetragen, wobei darauf geachtet wird, daß eine homogene Verteilung der Feststoffe in der Lipogelmatrix erfolgt.

### Anwendung

25 g der vorstehenden Blondiermittelsuspension werden mit 25 g einer 9prozentigen wasserstoffperoxidhaltigen Öl-in-Wasser-Emulsion der folgenden Zusammensetzung

| | |
|---|---|
| 9,0 g | Wasserstoffperoxid |
| 2,0 g | Cetylstearylalkohol |
| 0,2 g | Lanolinalkohol |
| 0,1 g | Phosphorsäure (85-prozentig) |
| 88,7 g | Wasser |
| 100,0 g | |

in einer Schale mit einem Pinsel homogen verrührt.
Das so erhaltene, breiartige Blondiermittel wird auf mittelbraunes Haar gleichmäßig aufgetragen und nach einer Einwirkungszeit von 30 Minuten bei Raumtemperatur mit warmem Wasser abgespült und getrocknet. Das so behandelte Haar ist auf einen hellblonden Farbton aufgehellt worden.

### Beispief 3: Blondiermittel

| Cremeförmige Blondiermittelsuspension | |
|---|---|
| 40,0 g | Octylstearat |
| 2,5 g | Paraffin Perliquidum |
| 2,0 g | Vaseline |
| 3,5 g | Aluminiumtristearat |
| 2,0 g | Jojobaöl |
| 4,0 g | Dinatriumpersulfat |
| 17,0 g | Dikaliumpersulfat |
| 8,0 g | Diammoniumpersulfat |
| 18,0 g | Natriummetasilikat |
| 2,5 g | Natriumalginat |
| 0,5 g | Ethylendiamintetraessigsäure-Dinatriumsalz |
| 100,0 g | |

Zur Herstellung dieser cremeförmigen Blondiermittelsuspension wird das Aluminiumtristearat im lipophilen Gemisch Octylstearat, Paraffinöl und Vaseline unter Erhitzen auf 110 bis 120°C vollständig aufgelöst. Das Jojobaöl wird bei 70°C homogen im abkühlenden Oleogel verteilt. Die vorgemischten festen Rohstoffe werden anschließend in das nach Abkühlen auf Raumtemperatur entstandene Lipogel eingetragen, wobei darauf geachtet wird, daß eine homogene Verteilung der Feststoffe in der Lipogelmatrix erfolgt.

### Anwendung

25 g der vorstehend beschriebenen Blondiermittelsuspension werden mit 37,5 g einer 6prozentigen wäßrigen Wasserstoffperoxidlösung in einer Schale mit einem Pinsel homogen verrührt. Es ist jedoch auch möglich, die Wasserstoffperoxidlösung in einer Auftrageflasche vorzulegen und mit der Blondiermittelsuspension zum gebrauchsfertigen Blondiermittel anzuschütteln. Das Blondiermittel wird gleichmäßig auf das aufzuhellende Haar aufgetragen und nach einer Einwirkungszeit von 40 Minuten bei Raumtemperatur mit Wasser abgespült. Sodann wird das Haar getrocknet. Der Aufhellungsgrad beträgt etwa 4 Tonstufen.

Alle Prozentangaben stellen, soweit nicht anders angegeben, Gewichtsprozente dar.

## Patentansprüche

1. Mittel zum Entfärben oder Blondieren von Haaren, welches unmittelbar vor der Anwendung mit einer wäßrigen Oxidationsmittelzubereitung vermischt wird und **dadurch gekennzeichnet ist, daß** es in Form einer Blondiermittelsuspension vorliegt und eine Kombination aus
(a) 20 bis 50 Gewichtsprozent mindestens einer organisch lipophilen Verbindung aus der Gruppe der pflanzlichen und tierischen Fette, Öle und Wachse, der Paraffinkohlenwasserstoffe, der höheren Alkohole und Ether, der aliphatischen und aromatischen Ester sowie der Silikonöle;
(b) 0,1 bis 40 Gewichtsprozent mindestens eines anorganischen oder organischen Verdickers mit lipophilem Charakter, der mit der lipophilen Verbindung ein Oleogel bzw. Lipogel ausbildet, welcher ausgewählt ist aus Erdalkallcarboxylaten, Aluminiumcarboxylaten, Copolymerisaten von Alkenen, vernetzten organischen Polymeren und lipophilisierten Schichtsilikaten oder Mischungen dieser Verdicker;
(c) 0,1 bis 40 Gewichtsprozent mindestens eines anorganischen oder organischen Verdickers mit hydrophilem Charakter, welcher ausgewählt ist aus Polymeren aus der Gruppe der Cellulosen, Alginate, Polysaccharide und Acrylsäuren;
(d) 10 bis 55 Gewichtsprozent mindestens eines anorganischen Persalzes;
(e) 15 bis 35 Gewichtsprozent mindestens eines alkalisch reagierenden Salzes;
sowie gegebenenfalls Hilfsstoffen und Zusatzstoffen enthält, und einen Wassergehalt von maximal 3% aufweist.

2. Mittel nach Anspruch 1, **dadurch gekennzeichnet, daß** die organisch lipophile Verbindung ausgewählt ist aus Pflanzenölen; Vaseline; flüssigen Paraffinen; Siliconölen: flüssigen, langkettigen, hydrophoben Fettsäureestern: natürlichern oder synthetischem Bienenwachs; C18- bis C36-Fettsäuren; C-10 bis C36-Fettsäuretriglyceriden und Fettsäureestergemischen oder Mischungen dieser Verbindungen.

3. Mittel nach Anspruch 2, **dadurch gekennzeichnet, daß** die organisch lipophile Verbindung ausgewählt ist aus Jojobaöl, Fettsäureestern, Paraffinöl, Kombinationen aus Fettsaureestern und Paraffinöl sowie Kombinationen aus Fettsäureestern und/oder Paraffinöl mit Vaseline.

4. Mittel nach einem der Ansprüche 1 bis 3, **dadurch gekennzeichnet, daß** der lipophile anorganische oder organische Verdicker ausgewählt ist aus Erdalkalistearaten, Aluminiumstearaten und Aluminium/ Magnesiumhydroxystearat.

5. Mittel nach einem der Ansprüche 1 bis 4, **dadurch gekennzeichnet, daß** der hydrophile anorganische oder organische Verdicker ausgewählt ist aus Methylcellulosen, Ethylcellulosen, Hydroxyethylcellulosen, Methylhydroxyethylcellulosen, Methylhydroxypropylcellulosen, Carboxymethylcellulosen, Alginsäure, Natriumalginat, Ammoniumalginat, Calciumalginat, Gummi Arabicum, Guar Gum, Xanthan Gum und Acrylsäurepolymeren mit einem Molekulargewicht von etwa 1.250.000 bis 4.000.000, oder Mischungen dieser Verdicker.

6. Mittel nach einem der Ansprüche 1 bis 5, **dadurch gekennzeichnet, daß** das anorganische Persalz ausgewählt ist aus Erdalkaliperoxiden, Ammoniumpersulfaten und Alkalipersulfaten.

7. Mittel nach einem der Ansprüche 1 bis 6, **dadurch gekennzeichnet, daß** das alkalisch reagierende Salz ausgewählt ist aus Natriumcarbonat; Natriumhydrogencarbonat, Magnesiumcarbonat, Ammoniumcarbonat, Ammoniumhydrogencarbonat und Natriumsilikaten oder Mischungen dieser Salze.

8. Mittel nach einem der Ansprüche 1 bis 7, **dadurch gekennzeichnet, daß** es frei von Tensiden ist

9. Mittel nach einem der Ansprüche 1 bis 8, **dadurch gekennzeichnet, daß** als wäßrige Oxidationsmittelzubereitung eine 6- bis 12-prozentige wäßrige Wasserstoffperoxidlösung oder Wasserstoffperoxidemulsion verwendet wird.

10. Mittel nach einem der Ansprüche 1 bis 9, **dadurch gekennzeichnet, daß** das Mischungsverhältnis von Blondiermittelsuspension zu Oxidationsmittelzubereitung 2:1 bis 1:8 beträgt.

11. Mittel nach einem der Ansprüche 1 bis 10, **dadurch gekennzeichnet, daß** das gebrauchsfertige Mittel zum Entfärben oder Blondieren von Haaren einen pH-Wert von 7,5 bis 11 aufweist.

## Claims

1. An agent for decolorizing or dyeing hair blond, which is mixed with an aqueous oxidizing agent preparation immediately prior to application and which is **characterized in that** it is available in the form of a bleaching agent suspension and features a combination of
(a) 20 to 50 percent by weight of at least one organic lipophilic compound in the vegetable and animal fats, oils and waxes group, the paraffin-hydrocarbons group, the higher alcohols and ethers group, the aliphatic and aromatic esters group and the silicone oils group;
(b) 0.1 to 40 percent by weight of at least one inorganic or organic thickener with lipophilic properties, which forms an oleogel or a lipogel with the lipophilic compound, which is selected from alkaline earth carboxylates, aluminum carboxylates, copolymerizates of alkenes, crosslinked organic polymers and lipophilized phylosilicates, or blends of these thickeners;
(c) 0.1 to 40 percent by weight of at least one inorganic or organic thickener with hydrophilic properties, which is selected from polymers in the cellulose, alginate, polysaccharide and acrylic acids group;
(d) 10 to 55 percent by weight of at least one inorganic persalt;
(e) 15 to 35 percent by weight of at least one alkaline salt;
and, if necessary, containing excipients and additives and with a maximum moisture content of 3%.

2. The agent according to claim 1, **characterized in that** the organic lipophilic compound is selected from vegetable oils; Vaseline; liquid paraffins; silicone oils; liquid, long-chain, hydrophobic fatty acid esters; natural or synthetic beeswax; C18 to C36 fatty acids; C10 to C36 fatty acid triglycerides and fatty acid ester compounds or blends of these compounds.

3. The agent according to claim 2, **characterized in that** the organic lipophilic compound is selected from jojoba oil, fatty acid esters, paraffin oil, combinations of fatty acid esters and paraffin oil, as well as combinations of fatty acid esters and/or paraffin oil with Vaseline.

4. The agent according to one of the claims 1 to 3, **characterized in that** the lipophilic inorganic or organic thickener is selected from alkaline earth stearates, aluminum stearates and aluminum / magnesium hydroxy stearate.

5. The agent according to one of the claims 1 to 4, **characterized in that** the hydrophilic inorganic or organic thickener is selected from methyl celluloses, ethyl celluloses, hydroxyethyl celluloses, methyl hydroxy ethyl celluloses, methyl hydroxy propyl celluloses, carboxy methyl celluloses, alginic acid, sodium alginate, ammonium alginate, calcium alginate, gum arabic, guar gum, xanthan gum and acrylic acid polymers with a molecular weight of approximately 1,250,000 to 4,000,000, or blends of these thickeners.

6. The agent according to one of the claims 1 to 5, **characterized in that** the inorganic persalt is selected from alkaline earth peroxides, ammonium persulfates and alkaline persulfates.

7. The agent according to one of the claims 1 to 6, **characterized in that** the alkaline salt is selected from sodium carbonate, sodium bicarbonate, magnesium carbonate, ammonium carbonate, ammonium bicarbonate and sodium silicates or blends of these salts.

8. The agent according to one of the claims 1 to 7, **characterized in that** it does not contain surfactants.

9. The agent according to one of the claims 1 to 8, **characterized in that** a 6 to 12% aqueous hydrogen peroxide solution or hydrogen peroxide emulsion is used as the aqueous oxidizing agent preparation.

10. The agent according to one of the claims 1 to 9, **characterized in that** the mixing ratio of bleaching agent suspension to oxidizing agent preparation is preferably 2:1 to 1:8.

11. The agent according to one of the claims 1 to 10, **characterized in that** the ready to use agent for decolorizing or dyeing hair blond has a pH of 7.5 to 11.

## Revendications

1. Agent destiné à décolorer ou colorer en blond les cheveux, qui est mélangé directement avant l'emploi avec une préparation aqueuse d'oxydant et est **caractérisé en ce qu'**il se trouve sous forme d'une suspension d'agent de coloration en blond et contient une association de
(a) 20 à 50 % en poids d'au moins un composé organique lipophile choisi dans le groupe des graisses, huiles et cires animales et végétales, des hydrocarbures paraffiniques, des alcools et éthers supérieurs, des esters aliphatiques et aromatiques ainsi que des huiles de silicone ;
(b) 0,1 à 40 % en poids d'au moins un épaississant minéral ou organique à caractère lipophile, qui forme un oléogel ou lipogel avec le composé lipophile et qui est choisi parmi les carboxylates de métaux alcalino-terreux, les carboxylates d'aluminium, les copolymères d'alcènes, les polymères organiques réticulés et les silicates lamellaires rendus lipophiles ou des mélanges de ces épaississants ;
(c) 0,1 à 40 % en poids d'au moins un épaississant minéral ou organique à caractère hydrophile, qui est choisi parmi les polymères du groupe des celluloses, alginates, polysaccharides et acides acryliques ;
(d) 10 à 55 % en poids d'au moins un persel minéral ;
(e) 15 à 35 % en poids d'au moins un sel à réaction alcaline ;
ainsi qu'éventuellement des additifs et adjuvants et présente une teneur en eau de maximum 3 %.

2. Agent selon la revendication 1, **caractérisé en ce que** le composé organique lipophile est choisi parmi les huiles végétales ; la vaseline ; les paraffines liquides ; les huiles de silicone ; les esters d'acides gras liquides, à longue chaîne, hydrophobes ; la cire d'abeille naturelle ou synthétique ; les acides gras en C 18 à C36 ; les triglycérides d'acides gras en C-10 à C36 et des mélanges d'esters d'acides gras ou des mélanges de ces composés.

3. Agent selon la revendication 2, **caractérisé en ce que** le composé organique lipophile est choisi parmi l'huile de jojoba, des esters d'acides gras, l'huile de paraffine, des associations d'esters d'acides gras et d'huile de paraffine ainsi que des associations d'esters d'acides gras et/ou d'huile de paraffine avec la vaseline.

4. Agent selon l'une des revendications 1 à 3, **caractérisé en ce que** l'épaississant minéral ou organique lipophile est choisi parmi les stéarates de métaux alcalino-terreux, les stéarates d'aluminium et l'hydroxystéarate d'aluminium/magnésium.

5. Agent selon l'une des revendications 1 à 4, **caractérisé en ce que** l'épaississant minéral ou organique lipophile est choisi parmi les méthylcelluloses, les éthylcelluloses, les hydroxyéthylcelluloses, les méthylhydroxyéthylcelluloses, les méthylhydroxypropylcelluloses, les carboxyméthylcelluloses, l'acide alginique, l'alginate de sodium, l'alginate d'ammonium, l'alginate de calcium, la gomme arabique, la gomme guar, la gomme xanthane et les polymères d'acide acrylique ayant une masse moléculaire d'environ 1 250 000 à 4 000 000 ou des mélanges de ces épaississants.

6. Agent selon l'une des revendications 1 à 5, **caractérisé en ce que** le persel minéral est choisi parmi les peroxydes de métaux alcalino-terreux, les persulfates d'ammonium et les persulfates de métaux alcalins.

7. Agent selon l'une des revendications 1 à 6, **caractérisé en ce que** le sel à réaction alcaline est choisi parmi le carbonate de sodium, l'hydrogénocarbonate de sodium, le carbonate de magnésium, le carbonate d'ammonium, l'hydrogénocarbonate d'ammonium et les silicates de sodium ou des mélanges de ces sels.

8. Agent selon une des revendications 1 à 7, **caractérisé en ce qu'**il est exempt de tensioactifs.

9. Agent selon une des revendications 1 à 8, **caractérisé en ce qu'**on utilise en tant que préparation aqueuse d'oxydant une solution aqueuse de peroxyde d'hydrogène ou émulsion aqueuse de peroxyde d'hydrogène à 6-12 %.

10. Agent selon une des revendications 1 à 9, **caractérisé en ce que** le rapport de mélange de la suspension d'agent de coloration en blond à la préparation d'oxydant va de 2:1 à 1:8.

11. Agent selon une des revendications 1 à 10, **caractérisé en ce que** l'agent pour la décoloration ou la coloration en blond des cheveux, prêt à l'emploi, présente un pH de 7,5 à 11.
